# EUROPEAN PATENT APPLICATION

(11) **EP 4 555 998 A1**
(43) Date of publication of application: **21.05.2025**
(21) Application number: 24213935.0
(22) Date of filing: 19.11.2024
(51) Int. Cl.: A61K 9/00, A61K 9/06, A61K 31/704, A61K 47/38, A61P 17/04, C08L 1/02

(54) **GLYCYRRHIZIC ACID BACTERIAL CELLULOSE COMPOSITE FOR TREATING ECZEMA IN SKIN**

(30) Priority: 20.11.2023 CN 202311555277
(71) Applicant: Chen, Chao-Cheng, 104 Taipei City (TW); Hong Qing Flowery Bio-tech (Guangzhou) Co., Ltd, Guangzhou City Guangdong (CN)
(72) Inventor: CHEN, Chao-Cheng, 104 Taipei City (TW); WANG, Qiong, Guangzhou City (CN); ZHENG, Si Han, Guangzhou City (CN)
(74) Representative: Epping - Hermann - Fischer

(57) **Abstract**

The present disclosure relates to a glycyrrhizic acid bacterial cellulose composite for treatment of eczema in skin, and the glycyrrhizic acid bacterial cellulose composite includes bacterial cellulose loaded with glycyrrhizic acid. The glycyrrhizic acid bacterial cellulose composite can be topically applied by transdermal application to skin, thereby achieving therapeutic effect by topical utilization on eczema in the skin.

## Description

### BACKGROUND

### 1. Technical Field

The present disclosure relates to a bacterial cellulose composite, especially to a glycyrrhizic acid bacterial cellulose composite and a method for preparing the same.

### 2. Description of Associated Art

Bacterial cellulose, a novel nanomaterial less applied in drug delivery, has been confirmed to have multiple special physical and chemical properties including biodegradability, nontoxic property, high elastic modulus, large specific surface area, low density, non-abrasiveness, ease to surface functionalization, high purity and crystallinity of chemical components, high degree of polymerization (2000 to 8000) and high hardness. Meanwhile, the bacterial cellulose is an inert material, and thus is less used directly in a study related to drug carriers. Glycyrrhizic acid is a drug commonly used in clinical and has effects of anti-inflammation and detoxication, while there is no practical application of glycyrrhizic acid for local administration.

Eczema, also known as atopic dermatitis or atopic eczema, is the greatest cause of disability due to skin diseases worldwide, affecting nearly 20% of children in the world. Eczema can be present in various forms, including atopic and non-atopic forms. The atopic form is usually mediated by IgE, and the non-atopic form is mediated by a non-IgE. Both forms can exhibit eosinophilia, and other variants of eczema include nodular eczema, seborrheic dermatitis and hand eczema. Eczema can also occur as a skin disease of other systemic diseases, such as Wiskott-Aldrich syndrome, human immunodeficiency virus (HIV) infection or food allergy. During the onset of eczema, excessive proliferation of keratinocytes and overexpression of inflammatory factors can be apparently observed in pathogenesis of eczema.

Therefore, there is an urgent need in the art for the treatment of eczema in clinical practice.

### SUMMARY

Given the various defects in prior art described above, the present disclosure provides a glycyrrhizic acid bacterial cellulose composite capable of treating eczema in skin and/or alleviating a symptom of eczema. The glycyrrhizic acid bacterial cellulose composite comprises bacterial cellulose loaded with glycyrrhizic acid, and the topical application of the glycyrrhizic acid bacterial cellulose composite can effectively treat eczema and alleviate the symptom of eczema significantly, which is beneficial to the treatment of eczema in skin, thereby promoting the regeneration of diseased region of eczema.

In an embodiment, the present disclosure provides a use of the glycyrrhizic acid bacterial cellulose composite aforementioned in manufacture of a medicament for treating eczema and/or alleviating a symptom of eczema. In another embodiment, the present disclosure also provides a method for treating eczema and/or alleviating a symptom of the eczema, including administering the glycyrrhizic acid bacterial cellulose composite, such as topically applying the glycyrrhizic acid bacterial cellulose composite to skin.

In an embodiment, the symptom includes a redness, an edema, an itching or any combination thereof.

In an embodiment, the bacterial cellulose is derived from *Acetobacterium Balch* and has a molecular weight of 50,000 to 2,500,000.

In an embodiment, the bacterial cellulose has 300 to 15,000 glucosyl groups.

In an embodiment, the mass ratio of the glycyrrhizic acid and bacterial cellulose is 1:0.1 to 10; preferably, the mass ratio of the glycyrrhizic acid and bacterial cellulose is 1:5.

The present disclosure further provides a method for preparing the glycyrrhizic acid bacterial cellulose composite, including steps: 1) mixing water and a lyophilized bacterial cellulose to obtain an aqueous bacterial cellulose; 2) dissolving glycyrrhizic acid in water to obtain an aqueous solution of glycyrrhizic acid; and 3) adding the aqueous solution of glycyrrhizic acid dropwise to the aqueous bacterial cellulose to obtain the glycyrrhizic acid bacterial cellulose composite.

In an embodiment, the aqueous bacterial cellulose and the aqueous solution of glycyrrhizic acid are each stirred to form homogeneous solutions.

In an embodiment, the stirred aqueous bacterial cellulose and the stirred aqueous solution of glycyrrhizic acid are each further subjected to an ultrasonic treatment.

In an embodiment, the adding is performed under ultrasonication.

In an embodiment, the lyophilized bacterial cellulose and water are mixed in a weight ratio of 1:10 to 15.

In an embodiment, the glycyrrhizic acid and water are mixed in a weight ratio of 1:0.5 to 1.5.

Specifically, the glycyrrhizic acid bacterial cellulose composite provided in the present disclosure is white sticky one and loaded with glycyrrhizic acid on the bacterial cellulose structure thereof, and can treat eczema in skin effectively.

The present disclosure also provides glycyrrhizic acid bacterial cellulose composites having different compositions, and the therapeutic effects of the composites onn the treatment of eczema in skin are compared. It can be seen from above, the glycyrrhizic acid bacterial cellulose composite provided in the present disclosure significantly inhibit the progression of lesions in the skin where eczema occurs and can regenerate the diseased region. In the present disclosure, a material having therapeutic effects on treating eczema in skin is obtained by an amphipathic property of the glycyrrhizic acid as well as a biocompatibility, a nontoxic property and a network structure of the bacterial cellulose. The most preferable dosages of the glycyrrhizic acid and bacterial cellulose can be further selected according to study result of facilitating the treatment of eczema in skin in combination with the requirements for a pharmaceutic formulation, and a related topical pharmaceutical formulation can be manufactured with a feed ratio achieving the best facilitation to the treatment of eczema in skin. Since the glycyrrhizic acid itself is an amphipathic compound, and the bacterial cellulose itself has properties including nontoxic property, high biocompatibility and utilization background as a cosmetic carrier, the present disclosure can be applied to the development study of a transdermal topical formulation for the treatment of eczema in skin and the facilitation of regeneration of skin where eczema occurs.

### BRIEF DESCRIPTION OF THE DRAWINGS

The embodiments of the present application will be illustrated by exemplary reference figures:
FIG. 1 shows SEM (Scanning Electron Microscope) images of Comparative Example 1, Comparative Example 3, and Example 5.
FIG. 2 is a bar graph showing scratching times of rats in Comparative Experimental Examples 1-5 and Experimental Examples 1-3.
FIGs. 3-5 show concentrations of different inflammatory indicators related to eczema in serum.
FIG. 6 shows images of skin tissues of SD rats stained with hematoxylin-eosin (HE staining) in Comparative Experimental Examples 1, 2 and 5 and Experimental Example 3 (i.e., the glycyrrhizic acid bacterial cellulose composite of Example 10).

### DETAILED DESCRIPTION OF THE PREFERRED EMBODIMENTS

The embodiments of the present application will be illustrated by the following embodiments, and those skilled in the art can easily understand the advantages and benefits of the present application from the content described in this specification. The present application can be practiced or applied by other different implementations, and different modifications and alternations can be made to the details of the present specification based on different views and applications without departing from the spirit described in the present application. Furthermore, all of the ranges and values mentioned herein are inclusive and combinable. Any numerical value or point, e.g., any integer, falling into a range described herein can be used as a lower or an upper limit to derive a subrange.

Glycyrrhizic acid, a drug commonly used in clinic, is capable of anti-inflammation and detoxication, which belongs to triterpenes and has amphipathic property, and thus exhibits the features of a surfactant. The aggregations or micella of glycyrrhizic acid can form an inclusion composite of host-guest with a hydrophobic drug, which can effectively increase the solubility of the drug and prevent the drug from setting down.

Bacterial cellulose possesses the features of biodegradability, nontoxic property, high elasticity modulus, high specific surface area, low density, non-abrasive, ease to surface functionality, high purity and crystallinity degree of chemical components, high degree of polymerization (2000 to 8000), high hardness, etc. However, the bacterial cellulose is very inert, and there is few related study in which the bacterial cellulose is applied as a topical pharmaceutical carrier for promoting the growth of hair.

Therefore, the present disclosure develops the bacterial cellulose as a topical pharmaceutical carrier by utilizing the amphipathic property of the glycyrrhizic acid, and it is shown by study that the glycyrrhizic acid bacterial cellulose composite can promote the growth and development of hair follicles in skin. As described above, in the present disclosure, the glycyrrhizic acid is used as a delivering drug, and bacterial cellulose is used as a carrier, so that the glycyrrhizic acid can be loaded in the network structure of the bacterial cellulose to form a new structure due to the amphipathic property of the glycyrrhizic acid, and the glycyrrhizic acid bacterial cellulose composite formed therefrom can significantly promote the growth and development of hair follicles in skin.

In one embodiment, the glycyrrhizic acid is loaded on the bacterial cellulose to form the glycyrrhizic acid bacterial cellulose composite.

In one embodiment, the bacterial cellulose is derived from *Acetobacterium Balch* bacterium and has a molecular weight (Mw) of 50,000 to 2,500,000, a molecular weight of 100,000 to 2,500,000, a molecular weight of 500,000 to 2,500,000, a molecular weight of 1,000,000 to 2,500,000, a molecular weight of 1,500,000 to 2,500,000, a molecular weight of 2,000,000 to 2,500,000, a molecular weight of 50,000 to 2,000,000, a molecular weight of 50,000 to 1,500,000, a molecular weight of 50,000 to 1,000,000, a molecular weight of 50,000 to 500,000, or a molecular weight of 50,000 to 100,000. For example, a molecular weight of 50,000, 100,000, 150,000, 200,000, 250,000, 300,000, 350,000, 400,000, 450,000, 500,000, 550,000, 600,000, 650,000, 700,000, 750,000, 800,000, 850,000, 900,000, 950,000, 1,000,000, 1,500,000, 2,000,000, or 2,500,000. In another embodiment, the bacterial cellulose is obtained from fermenting the *Acetobacterium Balch.*

In one embodiment, the bacterial cellulose has 300 to 15,000 glucosyl groups, 1,000 to 15,000 glucosyl groups, 2,000 to 15,000 glucosyl groups, 3,000 to 15,000 glucosyl groups, 4,000 to 15,000 glucosyl groups, 5,000 to 15,000 glucosyl groups, 6,000 to 15,000 glucosyl groups, 7,000 to 15,000 glucosyl groups, 8,000 to 15,000 glucosyl groups, 9,000 to 15,000 glucosyl groups, 10,000 to 15,000 glucosyl groups, 11,000 to 15,000 glucosyl groups, 12,000 to 15,000 glucosyl groups, 13,000 to 15,000 glucosyl groups, 14,000 to 15,000 glucosyl groups, 300 to 14,000 glucosyl groups, 300 to 13,000 glucosyl groups, 300 to 12,000 glucosyl groups, 300 to 11,000 glucosyl groups, 300 to 10,000 glucosyl groups, 300 to 9,000 glucosyl groups, 300 to 8,000 glucosyl groups, 300 to 7,000 glucosyl groups, 300 to 6,000 glucosyl groups, 300 to 5,000 glucosyl groups, 300 to 4,000 glucosyl groups, 300 to 3,000 glucosyl groups, 300 to 2,000 glucosyl groups, or 300 to 1,000 glucosyl groups, such as 300, 350, 400, 450, 500, 550, 600, 650, 700, 750, 800, 850, 900, 950, 1,000, 1,500, 2,000, 2,500, 3,000, 3,500, 4,000, 4,500, 5,000, 5,500, 6,000, 6,500, 7,000, 7,500, 8,000, 8,500, 9,000, 10,000, 10,500, 11,000, 11,500, 12,000, 12,500, 13,000, 13,500, 14,000, 14,500, or 15,000 glucosyl groups. Specifically, the bacterial cellulose has a chemical general formula of (C₆H₁₀O₅)ₙ, which is a polysaccharide consisting of a linear chain (glucoside bonds) of hundreds to thousands of β- (1→4)-linked D-glucose units. In other words, the bacterial cellulose is a macromolecular polysaccharide consisting of D-glucoses with 0-1,4-glucoside bonds.

In one embodiment, the mass ratio of the glycyrrhizic acid to the bacterial cellulose is 1: 0.1 to 10, e.g., 1: 0.1, 1: 0.11, 1: 0.13, 1: 0.14, 1: 0.17, 1: 0.2, 1: 0.3, 1: 0.33, 1: 0.4, 1: 0.5, 1: 0.6, 1: 0.7, 1: 0.8, 1: 0.9, 1: 1, 1: 2, 1: 3, 1: 4, 1: 5, 1: 6, 1: 7, 1: 8, 1: 9, or 1: 10. Preferably, the mass ratio of the glycyrrhizic acid to the bacterial cellulose is 1: 5.

The method for preparing the glycyrrhizic acid bacterial cellulose composite provided in the present disclosure includes the following steps: 1) mixing water and a lyophilized bacterial cellulose to obtain an aqueous bacterial cellulose; 2) dissolving glycyrrhizic acid in water to obtain an aqueous solution of glycyrrhizic acid; and 3) adding the aqueous solution of glycyrrhizic acid dropwise to the aqueous bacterial cellulose to obtain the glycyrrhizic acid bacterial cellulose composite.

In one embodiment, the lyophilized bacterial cellulose and water are mixed in a weight ratio of 1: 10 to 15, e.g., a weight ratio of 1: 10, 1: 11, 1: 12, 1: 13, 1: 14, or 1: 15.

In one embodiment,the glycyrrhizic acid and water are mixed in a weight ratio of 1: 0.5 to 1.5, e.g., 1: 0.5, 1: 0.6, 1: 0.7, 1: 0.8, 1: 0.9, 1: 1.0, 1: 1.1, 1: 1.2, 1: 1.3, 1: 1.4, or 1: 1.5.

### EXAMPLES

The present application will be illustrated in detail by specific examples below which should not to be considered to limit the scope of the present application.

### Preparation Example: glycyrrhizic acid bacterial cellulose composite

Glycyrrhizic acid (93%, G810520-25g, Shanghai Macklin Biochemical Technology Co., Ltd.) was added to distilled water, stirred to uniformity, and subjected to ultrasonication for 10 minutes to obtain an aqueous solution of glycyrrhizic acid. Meanwhile, the bacterial cellulose derived from *Acetobacterium Balch* (nanoscale lypophilized tablet of bacterial cellulose, Evophancie Biotech Ltd.) was added to distilled water, stirred thoroughly, and subjected to ultrasonication for 10 minutes to obtain an aqueous bacterial cellulose. Under ultrasonication, the aqueous solution of glycyrrhizic acid was added dropwise at a rate of 5 drops per 10 seconds to the aqueous bacterial cellulose, stirred thoroughly, and subjected to ultrasonication for 15 minutes to obtain the glycyrrhizic acid bacterial cellulose composite.

Following the method of the aforementioned Preparation Example, the bacterial cellulose composites of Examples 1-19 were prepared by using the composition in Table 1 below, respectively, and the mass ratios of the glycyrrhizic acid to the bacterial cellulose in the prepared glycyrrhizic acid bacterial cellulose composites (products) were listed.

**Table 1: Examples 1-19**

| | Aqueous solution of glycyrrhizic acid | | Aqueous bacterial cellulose | | Mass ratio of glycyrrhizic acid and bacterial cellulose in product |
|---|---|---|---|---|---|
| | Glycyrrhizic acid (mg) | Distilled water (mL) | Bacterial cellulose (mg) | Distilled water (mL) | |
| Example 1 | 10 | 0.8 | 10 | 0.13 | 1:1 |
| Example 2 | 10 | 0.8 | 20 | 0.26 | 1:2 |
| Example 3 | 10 | 0.8 | 30 | 0.39 | 1:3 |
| Example 4 | 10 | 0.8 | 40 | 0.52 | 1:4 |
| Example 5 | 10 | 0.8 | 50 | 0.65 | 1:5 |
| Example 6 | 10 | 0.8 | 60 | 0.78 | 1:6 |
| Example 7 | 10 | 0.8 | 70 | 0.91 | 1:7 |
| Example 8 | 10 | 0.8 | 80 | 1.04 | 1:8 |
| Example 9 | 10 | 0.8 | 90 | 1.17 | 1:9 |
| Example 10 | 10 | 0.8 | 100 | 1.3 | 1:10 |
| Example 11 | 100 | 8 | 50 | 0.65 | 2:1 |
| Example 12 | 150 | 12 | 50 | 0.65 | 3:1 |
| Example 13 | 200 | 16 | 50 | 0.65 | 4:1 |
| Example 14 | 250 | 20 | 50 | 0.65 | 5:1 |
| Example 15 | 300 | 24 | 50 | 0.65 | 6:1 |
| Example 16 | 350 | 28 | 50 | 0.65 | 7:1 |
| Example 17 | 400 | 32 | 50 | 0.65 | 8:1 |
| Example 18 | 450 | 36 | 50 | 0.65 | 9:1 |
| Example 19 | 500 | 40 | 50 | 0.65 | 10:1 |

### Comparative Example 1: Aqueous solution of glycyrrhizic acid

10 mg of glycyrrhizic acid (93%, G810520-25g, Shanghai Macklin Biochemical Technology Co., Ltd.) was added to 16 mL of distilled water, evenly stirred, and subjected to ultrasonication for 10 minutes to obtain an aqueous solution of glycyrrhizic acid.

### Comparative Example 2: Aqueous bacterial cellulose

50 mg of the bacterial cellulose derived from *Acetobacterium Balch* (nanoscale lypophilized tablet of bacterial cellulose, Evophancie Biotech Ltd.) was added to 1.3 mL of distilled water, stirred thoroughly, and subjected to ultrasonication for 10 minutes to obtain an aqueous bacterial cellulose.

### Comparative Example 3: Baicalin bacterial cellulose

10 mg of baicalin (8802695-5g, Shanghai Macklin Biochemical Technology Co., Ltd.) was added to 0.8 mL of distilled water, stirred thoroughly, and subjected to ultrasonication for 10 minutes to obtain an aqueous solution of baicalin. Meanwhile, 100 mg of the bacterial cellulose derived from *Acetobacterium Balch* (nanoscale lypophilized tablet of bacterial cellulose, Evophancie Biotech Ltd.) was added to 1.3 mL of distilled water, stirred thoroughly, and subjected to ultrasonication for 10 minutes to obtain aqueous bacterial cellulose. Under ultrasonication, the aqueous solution of baicalin was added to the aqueous bacterial cellulose, stirred thoroughly, and subjected to ultrasonication for 15 minutes. The baicalin bacterial cellulose composite is obtained after rotary evaporation.

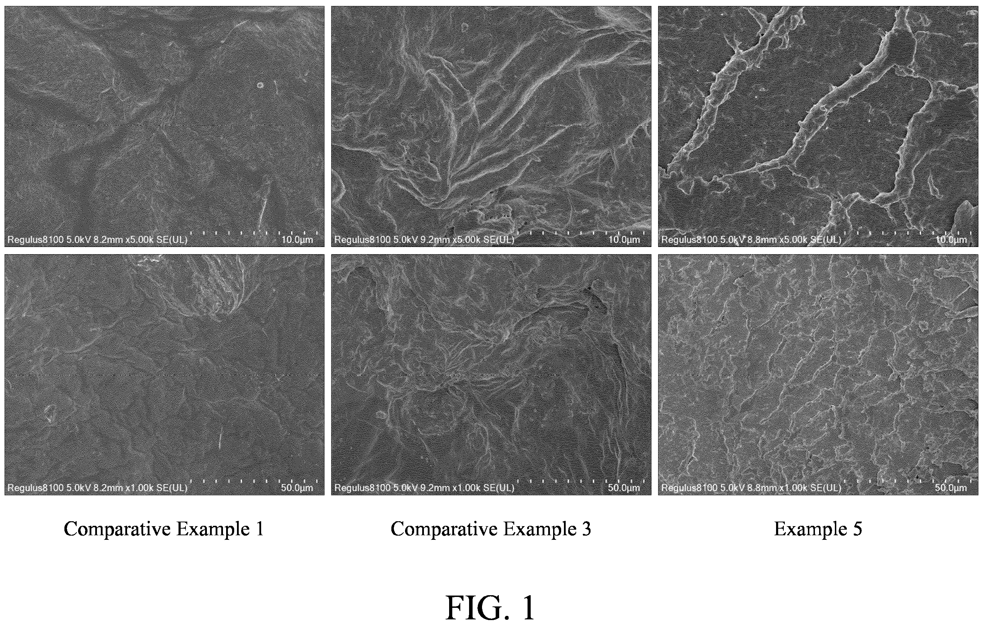

Please refer to FIG. 1. Comparative Example 1, Comparative Example 3, and Example 5 were determined by a scanning electron microscope (FEI Quanta 400 FEI, America FEI scanning electron microscope), respectively, and the resulting SEM images were shown in FIG. 1. As can be seen from FIG. 1, only the glycyrrhizic acid bacterial cellulose composite prepared in Example 5 had a structure of glycyrrhizic acid packaged in the network of the bacterial cellulose.

To test the therapeutic effect of different samples on eczema, SD rats were used to establish an eczema model, and untreated SD rats (i.e., blank group) were used as Comparative Experimental Example 1. Then, counting the writhing times of rats after applying each samples, detecting inflammatory indicators related to eczema in serum, and staining skin tissues with HE, as described in detail below.

### Comparative Experimental Example 2: rat models of DNCB-induced eczema

Before conducting the experiment, SD rats were subjected to hair removal in an area of 3 cm x 3 cm on back, 2% dinitrotoluene (DNCB) (Thermo Fisher Scientific Inc.) was applied on the hair-removed area at days 1 and 3 after initiation of the experiment, 0.5% DNCB was applied on day 6 of the experiment, thereafter, 0.5% DNCB was applied every 3 days (i.e., on days 9, 12, 15 and 18 of the experiment) to stimulate skin continuously, thereby rat models of DNCB-induced eczema were obtained.

### Comparative Experimental Example 3: rat models of eczema treated with the aqueous solution of glycyrrhizic acid

The rat models of eczema were obtained the same as that in Comparative Experimental Example 2, and Comparative Example 1 was administered on day 7 of the experiment.

### Comparative Experimental Example 4: rat models of eczema treated with aqueous bacterial cellulose

It was performed the same as Comparative Experimental Example 3, except that Comparative Example 1 administered on day 7 was replaced by Comparative Example 2.

### Comparative Experimental Example 5: rat models of eczema treated with a mometasone furoate cream

It was performed the same as Comparative Experimental Example 3, except that Comparative Example 1 administered on day 7 was replaced by a mometasone furoate cream (0.1% (5 g: 5 mg), 220908, Bayer Healthcare (Shanghai) Company Limited).

### Experimental Example 1: rat models of eczema treated with glycyrrhizic acid bacterial cellulose composite

It was performed the same as Comparative Experimental Example 3, except that Comparative Example 1 administered on day 7 was replaced by Example 1.

### Experimental Example 2: rat models of eczema treated with glycyrrhizic acid bacterial cellulose composite

It was performed the same as Comparative Experimental Example 3, except that Comparative Example 1 administered on day 7 was replaced by Example 5.

### Experimental Example 3: rat models of eczema treated with glycyrrhizic acid bacterial cellulose composite

It was performed the same as Comparative Experimental Example 3, except that Comparative Example 1 administered on day 7 was replaced by Example 10.

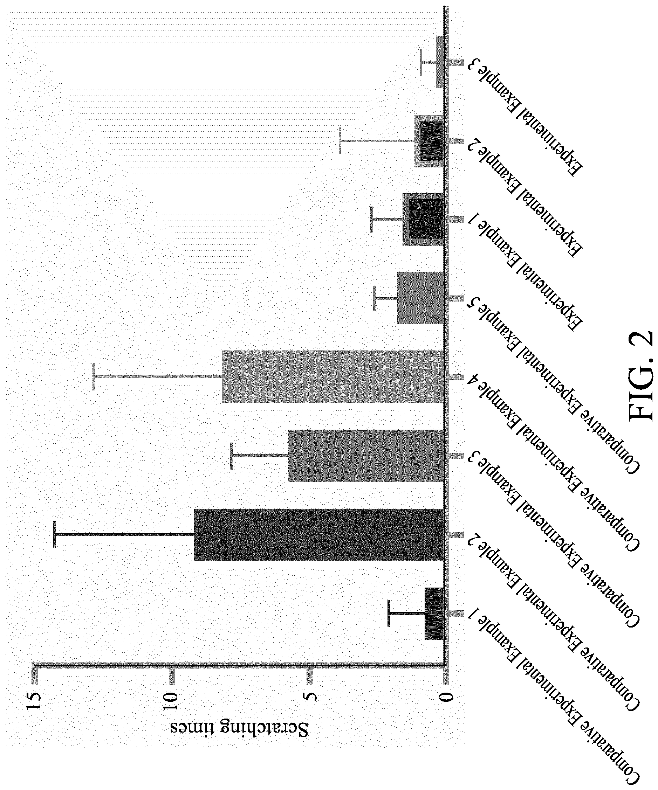

At day 18 of the experiment, 30 minutes after the administration of the samples, the scratching times (writhing times) of rats over 20 minutes were counted, and the results were shown in FIG. 2. The administration of the glycyrrhizic acid bacterial cellulose composite in Example 1, 5 and 10 arrived at itching-relieving effects in skin having eczema, and had the effects comparable to or even better than those of existing drug (i.e., Comparative Experimental Example 5) for treating eczema.

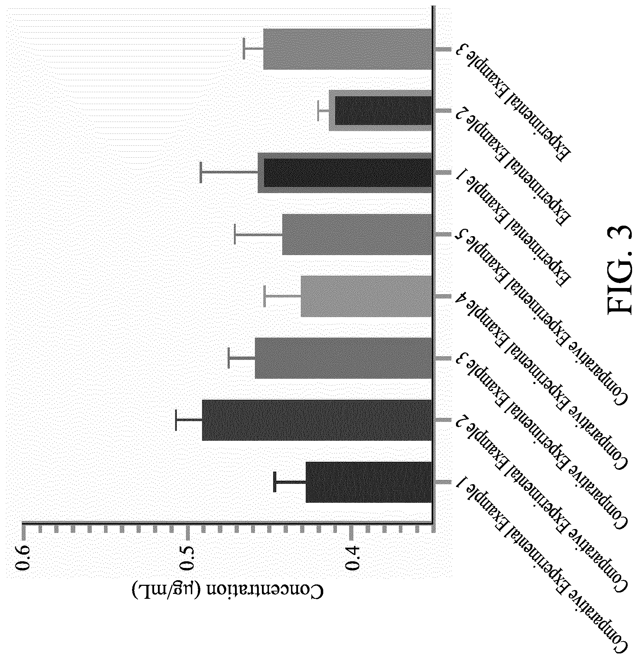

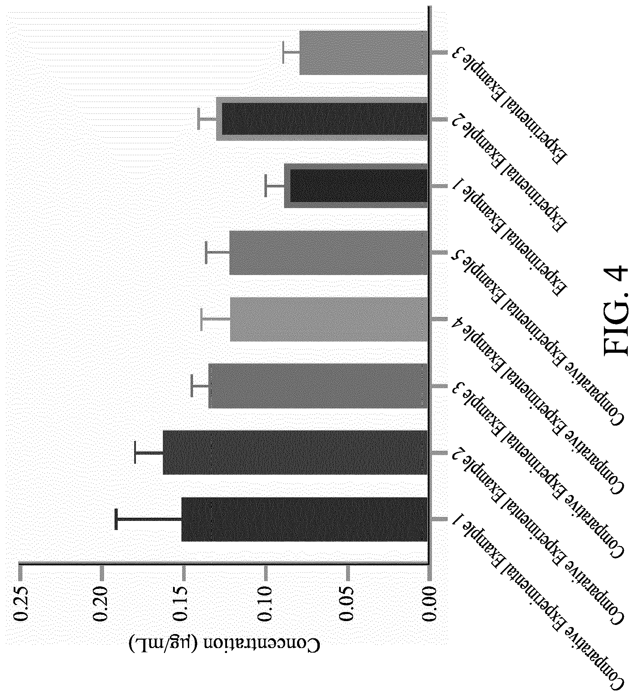

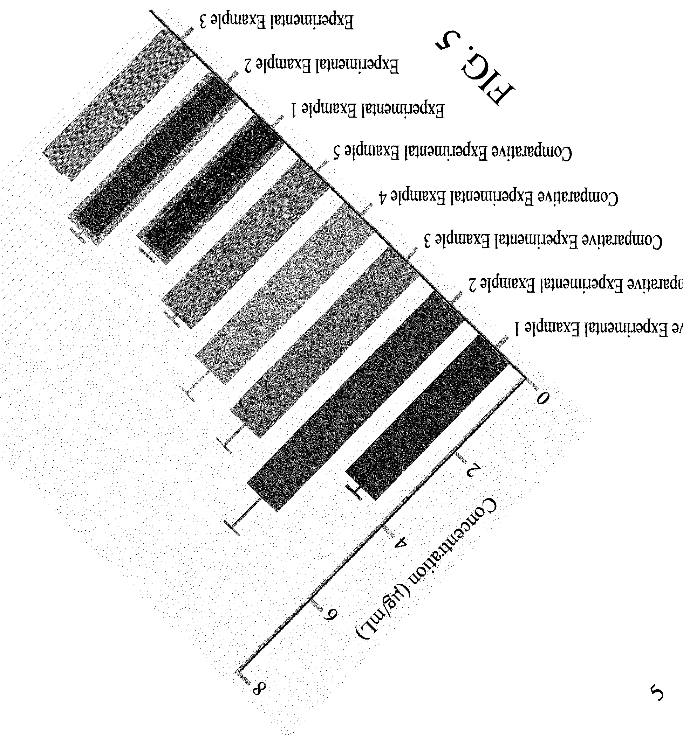

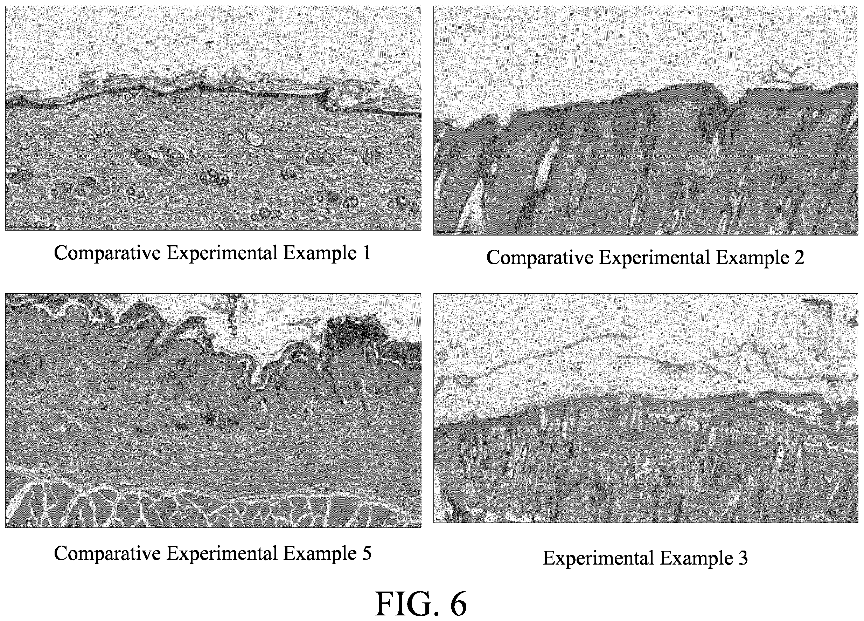

The rats were sacrificed after the experiment was conducted 19 days, and skins in the hair loss area and sera were collected for HE staining and detection of inflammatory indicators. Please refer to FIGs. 3-5 showing the concentrations of inflammatory indicators relevant with eczema in serum, i.e., immunoglobulin E (IgE), interleukin-4 (IL-4) and histamine, respectively, detected by an enzyme-linked immunosorbent assay (ELISA). As shown in FIGs. 3-5, the administration of the glycyrrhizic acid bacterial cellulose composites according to Examples 1, 5 and 10 significantly reduced the inflammatory indicators of IgE, IL-4 and histamine, which indicated occurrence of eczema. In addition, FIG. 6 showed the HE stained images of skins of SD rats to compare the effects on skins applied with different samples. As shown in FIG. 6, the area of skin administered with the glycyrrhizic acid bacterial cellulose composite of Example 10 had a dense skin layer with increased thickness of the epidermal layer.

In conclusion, the glycyrrhizic acid bacterial cellulose composite of the present disclosure has glycyrrhizic acid in the 3-dimensional network structure of bacterial cellulose, which can effectively treat eczema in skin and alleviate the symptoms thereof. In addition, the glycyrrhizic acid bacterial cellulose composite provided in the present disclosure is easy for manufacturing, the ratio of glycyrrhizic acid to bacterial cellulose contained in the composite can be easily adjusted, and is in prospect of applying.

The examples described above are provided for the purpose of illustration only and not for limiting the present application. Modifications and alternations can be made to the examples described above by one skilled in the art without departing from the spirit and scope of the present application. Therefore, the claimed range of the present application is defined by the claims attached, and should be encompassed in the technical solutions of the present disclosure as long as it has no impact on the results and implementation of the present application.

## Claims

1. A glycyrrhizic acid bacterial cellulose composite for treating eczema and/or alleviating a symptom of the eczema, comprising bacterial cellulose loaded with glycyrrhizic acid.

2. The glycyrrhizic acid bacterial cellulose composite of claim 1, wherein the symptom comprises a redness, an edema, an itching or any combination thereof.

3. The glycyrrhizic acid bacterial cellulose composite of claim 1, wherein the bacterial cellulose is derived from *Acetobacterium Balch* and has a molecular weight of 50,000 to 2,500,000.

4. The glycyrrhizic acid bacterial cellulose composite of claim 1, wherein the bacterial cellulose has 300 to 15,000 glucosyl groups.

5. The glycyrrhizic acid bacterial cellulose composite of claim 1, wherein a mass ratio of the glycyrrhizic acid to the bacterial cellulose is 1 :0.1 to 10.

6. The glycyrrhizic acid bacterial cellulose composite of claim 5, wherein the mass ratio of the glycyrrhizic acid to the bacterial cellulose is 1:5.

7. The glycyrrhizic acid bacterial cellulose composite of claim 1, wherein a method for preparing the glycyrrhizic acid bacterial cellulose composite comprises steps of:
mixing water and a lyophilized bacterial cellulose to obtain an aqueous bacterial cellulose;
dissolving glycyrrhizic acid in water to obtain an aqueous solution of glycyrrhizic acid; and
adding the aqueous solution of glycyrrhizic acid dropwise to the aqueous bacterial cellulose to obtain the glycyrrhizic acid bacterial cellulose composite.

8. The glycyrrhizic acid bacterial cellulose composite of claim 7, wherein the aqueous bacterial cellulose and the aqueous solution of glycyrrhizic acid are each stirred to form homogeneous solutions.

9. The glycyrrhizic acid bacterial cellulose composite of claim 7, wherein the stirred aqueous bacterial cellulose and the stirred aqueous solution of glycyrrhizic acid are each further subjected to an ultrasonic treatment.

10. The glycyrrhizic acid bacterial cellulose composite of claim 7, wherein the adding is performed under ultrasonication.

11. The glycyrrhizic acid bacterial cellulose composite of claim 7, wherein the lyophilized bacterial cellulose and water are mixed in a weight ratio of 1:10 to 15.

12. The glycyrrhizic acid bacterial cellulose composite of claim 7, wherein the glycyrrhizic acid and water were mixed in a weight ratio of 1:0.5 to 1.5.
